# EUROPEAN PATENT SPECIFICATION

(11) **EP 2 632 552 B1**
(45) Date of publication and mention of the grant of the patent: **27.11.2019**
(21) Application number: 11782318.7
(22) Date of filing: 28.10.2011
(51) Int. Cl.: A61P 17/00, A61P 31/10, A61K 8/49, A61K 31/555, A61Q 5/00, A61K 31/00

(54) **INHIBITION OF MICROBIAL GROWTH BY ACONITASE INHIBITION**
HEMMUNG DES MIKROBIELLEN WACHSTUMS MITTELS ACONITASEHEMMUNG
INHIBITION D'UNE CROISSANCE MICROBIENNE PAR INHIBITION DE L'ACONITASE

(30) Priority: 28.10.2010 US 407757 P
(43) Date of publication of application: 04.09.2013
(73) Proprietor: The Procter & Gamble Company, Cincinnati, OH 45202 (US)
(72) Inventor: SCHWARTZ, James, Robert, West Chester Ohio 45069 (US); SAUNDERS, Charles, Winston, Fairfield Ohio 45014 (US); YOUNGQUIST, Robert, Scott, Mason Ohio 45040 (US)
(74) Representative: Mathys & Squire
(86) International application number: PCT/US2011/058269
(87) International publication number: WO 2012/058526

(56) References cited:
- EP-A2- 0 422 508
- EP-A2- 0 800 814
- WO-A1-01/00151
- WO-A1-03/088965
- WO-A1-2006/110386
- JP-A- 2002 363 077
- LUSHCHAK O V ET AL: "Sodium nitroprusside induces mild oxidative stress in Saccharomyces cerevisiae", REDOX REPORT 2008 GB LNKD- DOI:10.1179/135100008X308885, vol. 13, no. 4, 2008, pages 144-152, XP009154992, ISSN: 1351-0002

## Description

### FIELD OF THE INVENTION

The present invention is directed to the inhibition of aconitase activity of fungal cells in an individual, comprising administering an inhibitor of aconitase activity to the fungal cell in an amount effective to inhibit activity of aconitase by said fungal cells.

### BACKGROUND OF THE INVENTION

Without being bound by theory, it is believed that the anti-fungal mechanism of action of pyrithione-containing antifungals involves the inhibition of aconitase and other mitochondrial iron-sulfur proteins. The mechanism of inhibition is believed to involve the formation of copper pyrithione within the cell by either association with exogenous or endogenous copper and transchelation according to the Irving-Williams series. The copper pyrithione can then traverse the mitochondrial membrane and interact with iron-sulfur proteins such as aconitase resulting in alteration of the iron-sulfur active site and resultant inhibition of activity.

In the present invention it was found that the ZPT sensitivity increased with a *CUP2* deletion strain that is defective in protection from high copper levels. From this and other observations, it is appreciated that copper is augmenting the effect of ZPT induced growth inhibition, but the mechanism was unknown.

Through the present invention, including the results of ZPT sensitivity of the *Saccharomyces* deletion library strains, it is found that mitochondrial iron sulfur protein maturation is a key target of ZPT. This is confirmed by the present invention's demonstration that, in minimal medium, ZPT inhibits growth by inhibition of glutamate and lysine synthesis, which require the activity of the mitochondrial iron-sulfur protein aconitase. Moreover, culturing of cells in the presence of ZPT has led to aconitase inhibition. The present invention has extended the understanding of the ZPT mechanism of action to identification of a key target enzyme.

International Patent Application No.: WO 03/088965 A1 relates to methods for delivering excess zinc to eukaryotic cells to inhibit the cell metabolism. It also relates to methods of treating microbial infections on the skin or scalp and to methods for the treatment of dandruff, and compositions which provide improved antidandruff activity.

European Patent Application No.: EP 0 422 508 A2 relates to a detergent composition comprising (a) 0.1 to 95% by weight of a saccharide nonionic surfactant and (b) 0.01 to 5% by weight of an antibacterial agent. The detergent compositions can effectively restrain the early occurrence of dandruff after shampooing when used as detergents for the scalp and the hair.

European Patent Application No.: EP 0 800 814 A2 relates to an antimicrobial hair treatment which is a shampoo composition comprising an antimicrobial compound selected from certain 1-hydroxy-6-substituted pyridine-2-one and 2-thione compounds, a shampoo surfactant and a polymeric, watersoluble cationic deposition aid. The pH of the hair treatment composition is adjusted to less than 7, so that the antimicrobial compound is present as insoluble particles in the hair treatment composition. In this way, enhanced antidandruff activity can be obtained from a rinse-off shampoo composition by utilising the antimicrobial compound in insoluble form and in combination with a cationic deposition polymer as a deposition aid for the insoluble particles of antimicrobial compound.

### SUMMARY OF THE INVENTION

The present invention is directed to an inhibitor of aconitase activity and a personal care composition comprising an inhibitor of aconitase activity for use in a method of treating fungi-mediated conditions or for use as an antifungal agent, as defined by the appended claims. The invention is further directed to a non-therapeutic use of an aconitase activity inhibitor for inhibiting aconitase activity in a fungal cell.

### DETAILED DESCRIPTION OF THE INVENTION

While the specification concludes with claims which particularly point out and distinctly claim the invention, it is believed the present invention will be better understood from the following description.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore do not include carriers or by-products that may be included in commercially available materials.

The components and/or steps, including those which may optionally be added, of the various embodiments of the present invention, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more".

Herein, "comprising" means that other steps and other ingredients which do not affect the end result can be added. This term encompasses the terms "consisting of' and "consisting essentially of'. The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

Herein, "aconitase inhibition" means a reduction in aconitase activity that can be recovered from lysed cells. Such aconitase inhibition may include direct inhibition of the enzyme or prevention of synthesis of active aconitase. Synthesis of active aconitase may be due to the lack of synthesis of aconitase protein, the lack of proper folding of the aconitase protein, the lack of incorporation of a functional iron-sulfur cluster into aconitase, or the presence of a damaged iron-sulfur cluster in aconitase.

Herein, "personal care composition" means products for and/or methods relating to treating hair (human, dog, and/or cat), including, bleaching, coloring, dyeing, conditioning, growing, removing, retarding growth, shampooing, styling; deodorants and antiperspirants; personal cleansing; color cosmetics; products, and/or methods relating to treating skin (human, dog, and/or cat), including application of creams, lotions, and other topically applied products for consumer use; and products and/or methods relating to orally administered materials for enhancing the appearance of hair, skin, and/or nails (human, dog, and/or cat); and shaving.

### A. Pyrithione or a Polyvalent metal salt of Pyrithione

In an embodiment, the present invention may comprise pyrithione or a polyvalent metal salt of pyrithione. Any form of polyvalent metal pyrithione salts may be used, including platelet and needle structures. In a further embodiment, salts for use herein include those formed from the polyvalent metals magnesium, barium, bismuth, strontium, copper, zinc, cadmium, zirconium and mixtures thereof, and in further embodiment, zinc. In yet a further embodiment, for use herein is the zinc salt of 1-hydroxy-2-pyridinethione (known as "zinc pyrithione" or "ZPT"); in another embodiment, ZPT in platelet particle form, wherein the particles have an average size of up to about 20µm, and in an embodiment have an average size of up to about 5µm, and yet in a further embodiment have an average size of up to about 2.5µm.

Pyridinethione anti-microbial and anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

It is further contemplated that when ZPT is used as the anti-microbial particulate in the anti-microbial compositions herein, that an additional benefit of hair growth or re-growth may be stimulated or regulated, or both, or that hair loss may be reduced or inhibited, or that hair may appear thicker or fuller.

Zinc pyrithione may be made by reacting 1-hydroxy-2-pyridinethione (i.e., pyrithione acid) or a soluble salt thereof with a zinc salt (e.g. zinc sulfate) to form a zinc pyrithione precipitate, as illustrated in U.S. Patent No. 2,809,971.

An embodiment of the present invention includes from about 0.01% to about 5% of a pyrithione or polyvalent metal salt of a pyrithione; and in a further embodiment from about 0.1% to about 2%.

### Method

Cultures of *Saccharomyces cerevisiae* strain BY4741 are grown in YPD (20 grams glucose, 20 grams peptone, 10 grams yeast extract per liter) broth overnight at 30°. The cultures are diluted with YPD to a total volume of 100 mls with an optical density (600 nm) of 0.1. The cultures are incubated with shaking until reaching an optical density of 0.2, whereupon the test material is added. The cultures are incubated overnight, and the cells are pelleted by centrifugation. The cell pellet is washed with 100 mM NaCl, 20 mM Tris pH 7.4 and resuspended in 5 ml of 100 mM NaCl, 20 mM Tris pH 7.4. Glass beads (0.5 mm) is added. The sample is alternately vortexed for one minute and incubated on ice for one minute, with a total of ten treatments. After centrifugation, the supernatant is collected. In a 96-well plate (UV-transparent Corning 3679 plate), 30 µl of cell lysate is mixed with 20 µl 100 mM NaCl, 20 mM Tris pH 7.4 and assayed using a Bioxytech Aconitase-340 kit (OxisResearch) according to instructions.

For the aconitase assay, samples are incubated for six minutes at 37°. The reaction rate is calculated based on the increase in optical density (340 nm) in the five-minute interval beginning one minute after the reaction started. The aconitase activity in lysates of treated samples is divided by the aconitase activity in lysates of untreated samples. If replicates were performed on samples, the reported data are a mean of the aconitase activity measurements of individual lysates of treated cells divided by the mean of the aconitase activity measurements of individual lysates of untreated cells. If the aconitase activity appears to be negative in lysates of treated cells, the value "0" is reported for aconitase activity (Table 1).
1,10-phenanthroline, N-hydroxy-6-octyloxypyridine-2(1H)one ethanolamine salt, piroctone olamine and 8-hydroxyquinoline are used for inhibiting aconitase activity according to the present invention, as defined in the appended claims.

**Table 1.**

| **Material** | **Concentration, ppm** | **Aconitase Activity, % of Untreated Control** |
|---|---|---|
| Zinc pyrithione (ZPT) | 5 | 0 |
| Copper Pyrithione (CuPT) | 5 | 0 |
| 1,10-Phenanthroline | 50 | 2 |
| N-Hydroxy-6-octyloxypyridine-2(1H)one, ethanolamine salt (1) | 50 | 0 |
| Piroctone Olamine (Octopirox) | 50 | 0 |
| 8-Hydroxyquinoline | 50 | 15 |
| EDTA | 50 | 36 |
| Zinc Sulfate | 50 | 110 |

| | | |
|---|---|---|
| (1) From Arch Chemicals, Inc. | | |

Desirable materials inhibit aconitase activity under the specified conditions by 70% or more (meaning in Table 1 residual activities of 30% or less). As demonstrated, both zinc and copper pyrithiones have the expected inhibition, as expected from the proposed mechanism of action. Other materials exemplified in Table 1 have affinity for metal ions as well, potentially influencing the iron-sulfur protein active site in a similar manner to the pyrithiones. For comparison, certain classical chelators, such as EDTA, are not effective, presumably due to poor cellular membrane permeation.

Aconitase inhibition has not previously been appreciated as a target for control of fungi. Thus, aconitase inhibitors such as the following list may have a function as anti-fungal;
Fluorocitrate - from fluoroacetate
   nitroisocitrate
   4-hydroxy-trans-aconitate
   oxalomalate (OMA, alpha-hydroxy-beta-oxalosuccinic acid)
ROS species or Oxidizing Species
   Peroxynitrite
   Nitric Oxide
   Hydrogen peroxide
   superoxide
Organic molecules from one paper
   1,2,3-D,L -Tricarboxycyclopentene-1
   Trimesic acid (1,3,5-tricarboxybenzene)
   Trimellitic acid (1,2,4-tricarboxybenzene)
   Pyromellitic acid (1,2,4,5-tetracarboxybenzene)
   1,2,3,4-Tetracarboxycyclopentane
Kreb Cycle Materials
   oxalosuccinate
   trans-aconitate
   cis-aconitate
   alpha-ketoglutaric
   2-oxoglutarate
   oxaloacetate
Others
   alloxan
   1-methyl-4-phenylpyridine
   Manganese
   Lon protease
   Deferiprone
   Pyrithione or metal salts of pyrithione
   Zinc pyrithione

N-Hydroxy-6-octyloxypyridine-2(1H)one, ethanolamine salt, (HP-101) as supplied from Arch Chemicals, Inc., is part of the N-Hydroxypyridones. The N-Hydroxypyridones have alkyl ether substitutions at the 6-position as free acids, ethanolamine salts and metal salts such as zinc, N-Hydroxy-6-octyloxypyridine-2(1H)one, zinc salt. The alkyl ether substituent is from 2-22 carbons in length, either linear or branched.

Further, iron-sulfur enzymes may be useful targets in a similar manner as is aconitase. Examples of such enzymes include biotin synthase, lipoic acid synthase, and homoaconitase.

In the present invention, an aconitase inhibitor may be used alone, or in combination with additional aconitase inhibitors and mixtures thereof. For example, zinc pyrithione may be used in combination with one or more additional aconitase inhibitors.

Disclosed herein is a method of inhibiting aconitase activity of fungal cells or microbial cells, the method comprising administering an inhibitor of aconitase activity, or an aconitase inhibitor to the fungal or microbial cell in an individual in an amount effective to inhibit activity of aconitase by said fungal cells.

Disclosed herein is a method of inhibiting aconitase activity of fungal cells or microbial cells, in an individual, the method comprising administering an inhibitor of aconitase activity, or an aconitase inhibitor to the fungal or microbial cell in an individual in an amount effective to inhibit activity of aconitase by said fungal cells.

In a further embodiment, the present invention comprises inhibiting aconitase activity of fungal cells wherein the individual is a dandruff sufferer.

Disclosed herein is a method of inhibiting aconitase activity of fungal cells wherein the inhibitor of aconitase is selected from the group consisting of fluorocitrate, 4-hydroxy-trans-aconitate, oxalomalate, alpha-hydroxy-beta-oxalosuccinic acid), ROS species or oxidizing Species, peroxynitrite, nitric oxide, hydrogen peroxide, superoxide, 1,2,3-D,L -tricarboxycyclopentene-1, trimesic acid (1,3,5-tricarboxybenzene), trimellitic acid (1,2,4-tricarboxybenzene), pyromellitic acid (1,2,4,5-tetracarboxybenzene), 1,2,3,4-tetracarboxycyclopentane, , kreb cycle materials, oxalosuccinate, trans-aconitate, cis-aconitate, alpha-ketoglutaric, 2-oxoglutarate, oxaloacetate, alloxan, 1-methyl-4-phenylpyridine, manganese, Lon protease, N-Hydroxy-6-octyloxypyridine-2(1H)one, ethanolamine salt pyrithione and metal salts of pyrithione, zinc pyrithione and mixtures thereof.

Disclosed herein is a method of inhibiting aconitase activity of fungal wherein the inhibitor of aconistase activity is zinc pyrithione.

In an embodiment, the present invention comprises inhibiting aconitase activity of fungal cells wherein the inhibitor of aconitase inhibits aconitase activity by at least 70%.

Disclosed herein is a method of inhibiting aconitase activity for an antifungal benefit comprising the steps of: a) exposing a cell to a material; b) opening of cell and measuring an aconitase activity and c) measuring the reduction in aconitase activity compared to a baseline or a control/untreated sample.

Disclosed herein is a method of inhibiting the growth of a fungal cell, said method comprising contacting said fungal cell with a material which inhibits aconitase activity by at least 70%, thereby causing an inhibition of growth of said fungal cell and/or causing death of the cell.

In a further embodiment, the present invention discloses a personal care composition comprising one or more of a material having inhibitory activity against an aconitase enzyme, as defined by the appended claims, wherein the material inhibits aconitase activity, by at least 70%, to provide enhanced efficacy to treat anti-fungal mediated conditions, such as anti-fungal mediated scalp conditions.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An inhibitor of aconitase activity which is selected from the group consisting of 1,10-phenanthroline and N-hydroxy-6-octyloxypyridine-2(1H)one ethanolamine salt for use in a method of treating fungi-mediated conditions by inhibiting aconitase activity of fungal cells in an individual by administering the inhibitor of aconitase activity to the fungal cell in an amount effective to inhibit activity of aconitase by said fungal cells.

2. The inhibitor of aconitase activity for use according to claim 1 wherein the individual is a dandruff sufferer.

3. The inhibitor of aconitase activity for use according to any preceding claims wherein the inhibitor of aconitase inhibits aconitase activity by at least 70%.

4. Non-therapeutic use of a material selected from the group consisting of 1,10-phenanthroline, N-hydroxy-6-octyloxypyridine-2(1H)one ethanolamine salt, piroctone olamine and 8-hydroxyquinoline for inhibiting aconitase activity in a fungal cell by at least 70% by contacting said fungal cell with the material, thereby causing an inhibition of growth of said fungal cell.

5. A personal care composition comprising one or more of a material having inhibitory activity against an aconitase enzyme, selected from the group consisting of 1,10-phenanthroline and N-hydroxy-6-octyloxypyridine-2(1H)one ethanolamine salt, wherein the material inhibits aconitase activity of fungal cells by at least 70%, for use in a method of treating fungi-mediated conditions in an individual.

6. A personal care composition for use according to claim 5, wherein the fungi-mediated conditions are fungi-mediated scalp conditions.

7. An inhibitor of aconitase activity which is selected from the group consisting of 1,10-phenanthroline and N-hydroxy-6-octyloxypyridine-2(1H)one ethanolamine salt for use as an antifungal agent.

8. A personal care composition comprising one or more of a material having inhibitory activity against an aconitase enzyme, selected from the group consisting of 1,10-phenanthroline and N-hydroxy-6-octyloxypyridine-2(1H)one ethanolamine salt, wherein the material inhibits aconitase activity by at least 70%, for use as an antifungal agent.

## Patentansprüche

1. Aconitase-Aktivitätshemmer, ausgewählt aus der Gruppe bestehend aus 1,10-Phenanthrolin und N-Hydroxy-6-octyloxypyridin-2(1H)on-ethanolaminsalz, zur Verwendung in einem Verfahren zum Behandeln von pilzvermittelten Leiden durch Hemmen der Aconitase-Aktivität von Pilzzellen bei einem Individuum durch Verabreichen des Aconitase-Aktivitätshemmers an die Pilzzelle in einer Menge, die wirksam ist, um die Aktivität der Aconitase durch die Pilzzellen zu hemmen.

2. Aconitase-Aktivitätshemmer zur Verwendung nach Anspruch 1, wobei das Individuum ein an Schuppen leidendes Individuum ist.

3. Aconitase-Aktivitätshemmer zur Verwendung nach einem der vorstehenden Ansprüche, wobei der Aconitase-Hemmer die Aconitase-Aktivität um mindestens 70 % hemmt.

4. Nicht-therapeutische Verwendung eines Materials, ausgewählt aus der Gruppe bestehend aus 1,10-Phenanthrolin, N-Hydroxy-6-octyloxypyridin-2(1H)on-ethanolaminsalz, Pirocton-Olamin und 8-Hydroxychinolin, zum Hemmen der Aconitase-Aktivität in einer Pilzzelle um mindestens 70 % durch Inkontaktbringen der Pilzzelle mit dem Material, wodurch eine Hemmung des Wachstums der Pilzzelle bewirkt wird.

5. Körperpflegezusammensetzung, umfassend ein oder mehrere aus einem Material mit hemmender Wirkung gegen ein Aconitase-Enzym, ausgewählt aus der Gruppe bestehend aus 1,10-Phenanthrolin und N-Hydroxy-6-octyloxypyridin-2(1H)on-ethanolaminsalz, wobei das Material die Aconitase-Aktivität von Pilzzellen um mindestens 70 % hemmt, zur Verwendung in einem Verfahren zum Behandeln von pilzvermittelten Leiden bei einem Individuum.

6. Körperpflegezusammensetzung zur Verwendung nach Anspruch 5, wobei es sich bei den pilzvermittelten Leiden um pilzvermittelte Kopfhautleiden handelt.

7. Aconitase-Aktivitätshemmer, ausgewählt aus der Gruppe bestehend aus 1,10-Phenanthrolin und N-Hydroxy-6-octyloxypyridin-2(1H)on-ethanolaminsalz, zur Verwendung als ein Antimykotikum.

8. Körperpflegezusammensetzung, umfassend ein oder mehrere aus einem Material mit hemmender Wirkung gegen ein Aconitase-Enzym, ausgewählt aus der Gruppe bestehend aus 1,10-Phenanthrolin und N-Hydroxy-6-octyloxypyridin-2(1H)on-ethanolaminsalz, wobei das Material die Aconitase-Aktivität um mindestens 70 % hemmt, zur Verwendung als Antimykotikum.

## Revendications

1. Inhibiteur de l'activité aconitase qui est choisi dans le groupe constitué de 1,10-phénanthroline et de sel d'éthanolamine de N-hydroxy-6-octyloxypyridine-2(1H)un pour une utilisation dans un procédé de traitement de pathologies médiées par des champignons en inhibant l'activité aconitase de cellules fongiques chez un individu en administrant l'inhibiteur d'activité aconitase à la cellule fongique en une quantité efficace pour inhiber l'activité de l'aconitase par lesdites cellules fongiques.

2. Inhibiteur de l'activité aconitase pour une utilisation selon la revendication 1 dans lequel l'individu est une personne souffrant de pellicules.

3. Inhibiteur de l'activité aconitase pour une utilisation selon l'une quelconque des revendications précédentes dans lequel l'inhibiteur d'aconitase inhibe l'activité aconitase d'au moins 70 %.

4. Utilisation non thérapeutique d'un matériau choisi dans le groupe constitué de 1,10-phénanthroline, de sel d'éthanolamine de N-hydroxy-6-octyloxypyridine-2(1H)un, de pirocton olamine et de 8-hydroxyquinoline pour inhiber l'activité aconitase dans une cellule fongique d'au moins 70 % en mettant en contact ladite cellule fongique avec le matériau, provoquant ainsi une inhibition de la croissance de ladite cellule fongique.

5. Composition de soins personnels comprenant un ou plusieurs d'un matériau ayant une activité inhibitrice contre une enzyme aconitase, choisi dans le groupe constitué de 1,10-phénanthroline et de sel d'éthanolamine de N-hydroxy-6-octyloxypyridine-2(1H)un, dans laquelle le matériau inhibe l'activité aconitase de cellules fongiques d'au moins 70 %, pour une utilisation dans un procédé de traitement de pathologies médiées par des champignons chez un individu.

6. Composition de soins personnels pour une utilisation selon la revendication 5, dans laquelle les pathologies médiées par les champignons sont des pathologies du cuir chevelu.

7. Inhibiteur d'activité aconitase qui est choisi dans le groupe constitué de 1,10-phénanthroline et de sel d'éthanolamine de N-hydroxy-6-octyloxypyridine-2(1H)un pour une utilisation comme agent antifongique.

8. Composition de soins personnels comprenant un ou plusieurs matériaux ayant une activité inhibitrice d'un enzyme aconitase, choisi dans le groupe constitué de 1,10-phénanthroline et de sel d'éthanolamine de N-hydroxy-6-octyloxypyridine-2(1H)un, dans laquelle le matériau inhibe l'activité aconitase d'au moins 70 %, pour une utilisation en tant qu'agent antifongique.
